# EUROPEAN PATENT SPECIFICATION

(11) **EP 1 181 002 B1**
(45) Date of publication and mention of the grant of the patent: **27.07.2005**
(21) Application number: 00935042.2
(22) Date of filing: 12.05.2000
(51) Int. Cl.: A61K 7/00, A61K 7/50

(54) **FOAMABLE SHOWER OIL COMPOSITION**
SCHÄUMBARE DUSCHÖL-ZUSAMMENSETZUNG
COMPOSITION HUILEUSE MOUSSANTE POUR LA DOUCHE

(30) Priority: 28.05.1999 GB 9912596; 08.11.1999 GB 9926453
(43) Date of publication of application: 27.02.2002
(73) Proprietor: UNILEVER PLC, London EC4P 4BQ (GB); UNILEVER N.V., 3013 AL Rotterdam (NL)
(72) Inventor: MEYER, Frank, Edgewater, NJ 07020 (US); BARSCHKE, Joern, 21614 Buxtehude (DE); DITMER, Monika, 21614 Buxtehude (DE)
(74) Representative: Mulder, Cornelis Willem Reinier, Dr.
(86) International application number: PCT/EP2000/004442
(87) International publication number: WO 2000/072805

(56) References cited:
- WO-A-92/11839
- WO-A-98/27936
- US-A- 5 002 680
- US-A- 5 286 475
- US-A- 5 653 988

## Description

The present invention relates to a packaged detergent product comprising an anhydrous foamable composition, preferably for use as a shower product. Conveniently, the packaged product is in the form of a propellant driven aerosol which dispenses the product in the form of a mousse.

It is desirable that skin cleansing compositions not only clean the body, but may also have a regenerating, normalising, skin caring and/or moisturising effect on the human skin, for example by including a benefit agent in the cleansing composition, such as a suitable emollient or oil.

Bath oil formulations comprising a mixture of surfactants and an oil component are known. By varying the concentration of the surfactant, the concentration of the oil component, the particular type of surfactant and/or the type of oil component in the composition, and by including other additives, such as foam improvers, it is possible to produce high-foaming, low-foaming or non-foaming cleansing compositions having skin care properties, as an oil film or an oil-in-water system is formed on the surface of the bath water.

A disadvantage with a bath oil formulations is that in use the appropriate dosage of the formulation is diluted several hundred fold, and the beneficial effects of the formulation are thereby reduced unless either a large quantity of the bath oil formulation is added to the bath, or a particularly complicated and concentrated bath oil formulation is used.

A further disadvantage of such products which often comprise for the majority part simply surfactant and hydrophobic component (eg. oil), is that they are not suitable for use as cleansing or moisturising compositions in a shower context. A particular disadvantage of such compositions, which typically comprise in the most part surfactant and oil, is that they can be difficult to manipulate.

Unlike a bath oil formulation, a cleansing formulation for use in the shower needs to have an increased viscosity, so that it does not run out of the users' hand after being dispensed from a container. Bath oil formulations are typically unsuitable for such use in the shower not only because of the difficulties associated with dosing and manipulation but also because such formulations do not produce the required amount of foam.

A further problem which has been found in such surfactant and oil compositions is that they are prone to oxidation, leading to a product which may relatively rapidly deteriorate after being opened, and hence is unsatisfactory and has too short a useful life from a consumer perspective.

Suitable cleansing products for use in the shower may also be relatively difficult to formulate in that aesthetically pleasing, stable compositions may not readily form, or if they do form they may be unstable.

US 5,002,680 describes an aerosol mousse forming emulsion comprising specified amounts of mild surfactant, polymeric skin fed aid, moisturiser and a balance of water as well as propellant.

The problem of providing a shower oil formulation which displays the required cleansing, foaming and skin caring action has long been recognised, because typically a shower formulation comprising a mixture of surfactants and a high oil component concentration exhibits low lathering.

For example, European Patent No. 0,691,127 relates to a cosmetic or dermatological shower preparation comprising not more than 55% by weight of a surface active substance and not less than 45% by weight of an oil component.

Accordingly, there is a need for providing a foamable shower oil product which not only has the required cleansing and foaming action, but also smoothes the skin, reduces the dryness of the skin and makes the skin supple.

Compositions according to the invention may also improve on one or more of the disadvantages of the prior art generally discussed above, including reduced oxidation and ease of handling, as well as providing satisfactory foaming.

According to a first aspect, the present invention provides a packaged product for providing a foamable shower oil formulation in the form of a mousse comprising:
a sealed container having a dispensing means; and
an anhydrous foamable cleansing and skin care composition in the sealed container, comprising by weight of the composition:-
   i. 35 to 40% of an oil component;
   ii. 45 to 60% of an anionic surfactant;
   iii. 4% to 15% of a propellant; and
   iv. 0 to 5% of a cosmetic or pharmaceutical adjunct; and
   v. less than 3% water.

Packaged compositions according to the invention are packaged in hardware containing a propellant gas, such that on dispensing the composition instantly forms a creamy foam. Such packaging and hardware is well known in the art.

Packaged compositions according to the invention have been found to have a number of associated advantages. The combination of the foamable shower oil composition packaged as a foamable concentrate in a sealed container having a dispensing means which forms a mousse on dispensing not only prevents oxidation and degradation of the shower oil composition during storage or standing, but also provides means for conveniently dispensing the required amount of the composition from the container.

Once dispensed, the product is more easily manipulated and distributed, compared to a low viscosity liquid.
Furthermore, although the shower oil composition is anhydrous, it exhibits surprisingly good foam production, high cleansing power, a high degree of skin regenerating action, and improved spreadability following dispersal from the pressurised container.

In particular, packaged compositions according to the invention have also been found to have surprisingly high degrees of foaming, considering that they are anhydrous. By "anhydrous" in the context is meant that the composition contains less than about 3% by weight of water, more preferably less than about 1% by weight. Ideally no water is present in the composition.

Compositions according to the invention contain an anionic surfactant. The surfactant is preferably one which is liquid at ambient temperature, or soluble in the non-oil components of the composition.

Suitable anionic surfactants are the alkyl sulphates, alkyl ether sulphates, alkaryl sulphonates, alkyl succinates, alkyl sulphosuccinates, N-alkoyl sarcosinates, and alphaolefin sulphonates, especially their sodium, magnesium, ammonium and mono-, di- and triethanolamine salts. The alkyl groups generally contain from 8 to 18 carbon atoms and may be unsaturated. The alkyl ether sulphates may contain from one to 10 ethylene oxide (EO) or propylene oxide units per molecule, and preferably contain 2 to 3 ethylene oxide units per molecule.

Examples of suitable anionic surfactants include sodium lauryl sulphate, sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. More preferably, the anionic surfactant is chosen from sodium lauryl sulphate, sodium lauryl ether sulphate 2EO and 3EO, ammonium lauryl sulphate and/or ammonium lauryl ether sulphate 1EO, 2EO and 3EO, acyl isethionates, and alkyl phosphates.

The nonionic surfactants suitable for use in the composition of the invention may include condensation products of aliphatic (C₈-C₁₈) primary or secondary linear or branched chain alcohols or phenols with alkylene oxides, usually ethylene oxide.

Other suitable nonionics include mono- or di-alkyl alkanolamides or alkyl polyglucosides. Examples include coco mono- or diethanolamide, coco mono isopropanolamide, and coco diglucoside. Preferably, the nonionic surfactant if present is used at a concentration of 0.1 to 10%, most preferably 0.1 to 3%, by weight of the composition.

The amphoteric surfactants suitable for use in the composition of the invention may include alkyl amine oxides, alkyl betaines, alkyl amidopropyl betaines, alkyl mono- or dialkanolamides, alkyl sulphobetaines, alkyl glycinates and alkyl carboxyglycinates, wherein the alkyl groups have from 8 to 18 carbon atoms. Examples include lauryl amine oxide, cocomonoethanolamide, cocodiethanolamide, cocamidopropyl betaine, cocodimethyl sulphopropyl betaine and cocobetaine. Preferably, if present the composition includes 0.1 to 50% by weight, preferably 1-30%, more preferably 1-20%, and preferably 1 to 10% by weight, of amphoteric surfactant.

Preferably the surfactant is a fatty alcohol ethoxylate, a fatty alcohol sulphate, a fatty alcohol sulphate amide or a fatty diethenolamide and mixtures thereof.

Compositions according to the invention also include an oil component. Preferred oil components are benefit agents, which may be solid or liquid at room temperature, but in compositions according to the invention are to be found in liquid form (either by virtue of being liquid at ambient temperature themselves, or by being solubilised in a hydrophobic liquid component so as to provide a liquid composition), include:
a) silicone oils, gums and modifications thereof such as linear and cyclic polydimethylsiloxanes, amino, alkyl alkylaryl and aryl silicone oils;
b) fats and oils including natural fats and oils such as jojoba, soyabean, rice bran, avocado, almond, olive, sesame, persic, castor, coconut, mink oils; cacao fat, beef tallow, lard; hardened oils obtained by hydrogenating the aforementioned oils; and synthetic mono, di and triglycerides such as myristic acid glyceride and 2-ethylhexanoic acid glyceride;
c) waxes such as carnauba, spermaceti, beeswax, lanolin and derivatives thereof;
d) hydrophobic plant extracts;
e) hydrocarbons such as liquid paraffins, petroleum jelly, microcrystalline wax, ceresin, squalene, squalane, and mineral oil;
f) higher fatty acids such as lauric, myristic, palmitic, stearic, behenic, oleic, linoleic linolenic, lanolic, isostearic and poly unsaturated fatty acids (PUFA) acids;
g) higher alcohols such as lauryl, cetyl, steryl, oleyl, behenyl, cholesterol and 2-hexadecanol alcohol;
h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate for example lauryl lactate, alkyl citrate and alkyl tartrate;
i) essential oils such as fish oils, mentha, jasmine, camphor,white cedar, bitter orange peel, ryu,turpentine, cinnamon, bergamont, citrus unshiu, calamus, pine, lavender, bay, clove, hiba, eucalyptus, lemon, starflower, thyme, peppermint, rose, sage, menthol, cineole, eugeriol, citral, citronelle, borneol, linalool, geraniol, evening primrose, camphor, thymol, spirantol, pinene, limonene and terpenoid oils;
j) lipids such as cholesterol, ceramides, sucrose esters and pseudo-ceramides as described in European Patent Specification No. 556 957;
k) vitamins such as vitamin A and E, and vitamin alkyl esters, including those vitamin C alkyl esters;
l) sunscreens such as octyl methoxyl cinnamate (Parsol MCX) and butyl methoxy benzoylmethane (Parsol 1789)
m) Phospholipids;
n) mixtures of any of the foregoing components.

Preferably, the oil component is a mineral oil, an animal oil, a vegetable oil, a synthetic oil, a triglyceride of a fatty acid and/or fatty alcohol, an ester of a fatty acid and/or a fatty alcohol or a mixture thereof.

Suitable cosmetic and/or pharmaceutical agents are those routinely used in the art, such as for example perfumes, dyestuffs, or antimicrobial agents. Compositions according to the invention are however washing compositions. Compositions according to the invention also utilise a propellant gas, which in combination with the appropriate packaging hardware generates a mousse on dispensing. The propellant may be any conventionally used propellant gas, which is either flammable or non-flammable. Conveniently the propellant gas is a short chain hydrocarbon, such as butane or isobutane. Conveniently the propellant is used at ratios of composition to propellant in the region 80:20 to 98:2, preferably in the region 85:15 to 92:8.

The following comparative compositions represent topical mousse compositions.

| Ingredient | %w/w | %w/w | %w/w |
|---|---|---|---|
| Anionic Sufactant (SLES.2EO) | 40 | 30 | 40 |
| Amphoteric Surfactant (Cocoamidopropyl betaine) | 20 | 27 | 20 |
| Soya oil | 35 | 40 | 37 |
| Perfume | 2 | 2 | 2 |
| Preservative (Vit. E acetate) | 1 | 1 | 1 |

Compositions according to the invention were found to have good foaming properties and improved ease of dispersion, better spreadability and better stability with regard to oxidation compared to similar compositions packaged in simple containers which were not pressurised aerosols.

## Claims

1. A packaged product for providing a foamable shower oil formulation in the form of a mousse comprising:
a sealed container having a dispensing means; and
an anhydrous foamable cleansing and skin-caring composition in the sealed container, comprising by weight of the composition:
i. 35 to 40% of an oil component;
ii. 45 to 60% of an anionic surfactant;
iii. 4 to 15% of a propellant;
iv. 0 to 5% of a cosmetic or pharmaceutical adjunct; and
v. less than 3% water

2. A packaged product as claimed in Claim 1 wherein the oil component is a mineral oil, an animal oil, a vegetable oil, a synthetic oil, a triglyceride of a fatty acid and/or a fatty alcohol, an ester of a fatty acid and/or a fatty alcohol, or a mixture thereof.

3. A packaged product according to claim 1 or claim 2, wherein the surfactant is selected from the group consisting of a fatty alcohol sulphate, a fatty alcohol sulphate amide, a fatty alcohol ether sulphate, and mixtures thereof.

## Patentansprüche

1. Abgepacktes Produkt zum Bereitstellen einer schäumbaren Duschölformulierung in der Form einer Mousse, umfassend: einen verschlossenen Behälter mit einer Dispenservorrichtung und eine wasserfreie schäumbare Reinigungs- und Hautpflegezusammensetzung in dem verschlossenen Behälter, umfassend, bezogen auf das Gewicht der Zusammensetzung:
i. 35 bis 40 % einer Ölkomponente;
ii. 45 bis 60 % eines anionischen oberflächenaktiven Stoffes;
iii. 4 bis 15 % eines Treibmittels;
iv. 0 bis 5 % eines kosmetischen oder pharmazeutischen Hilfsstoffes; und
v. weniger als 3 % Wasser.

2. Abgepacktes Produkt nach Anspruch 1, wobei die Ölkomponente ein Mineralöl, ein Tieröl, ein Pflanzenöl, ein synthetisches Öl, ein Triglycerid einer Fettsäure und/oder eines Fettalkohols, ein Ester einer Fettsäure und/oder eines Fettalkohols oder ein Gemisch davon ist.

3. Abgepacktes Produkt nach Anspruch 1 oder Anspruch 2, wobei der oberflächenaktive Stoff aus der Gruppe, bestehend aus einem Fettalkoholsulfat, einem Fettalkoholsulfatamid, einem Fettalkoholethersulfat und Gemischen davon, ausgewählt ist.

## Revendications

1. Produit conditionné permettant de fournir une formulation d'huile pour la douche capable de mousser sous la forme d'une mousse, comprenant:
un récipient scellé comprenant des moyens de distribution; et une composition nettoyante et soin de la peau anhydre et capable de mousser, placée dans le récipient scellé, comprenant sur la base du poids de la composition :
i. de 35 à 40 % d'un composant d'huile ;
ii. de 45 à 60 % d'un tensioactif anionique ;
iii. de 4 à 15 % d'un propulseur ; et
iv. de 0 à 5 % d'un additif cosmétique ou pharmaceutique ; et
v. moins de 3 % d'eau.

2. Produit conditionné selon la revendication 1, dans lequel le composant d'huile est une huile minérale, une huile animale, une huile végétale, une huile synthétique, un triglycéride d'un acide gras et/ou d'un alcool gras, un ester d'un acide gras et/ou d'un alcool gras, ou un mélange de ceux-ci.

3. Produit conditionné selon la revendication 1 ou la revendication 2, dans lequel le tensioactif est sélectionné à partir du groupe constitué d'un sulfate d'alcool gras, d'un sulfate amide d'alcool gras, d'un éther sulfate d'alcool gras et de mélanges de ceux-ci.
